# FASCICULE DE BREVET EUROPEEN

(11) **EP 2 101 669 B1**
(45) Date de publication et mention de la délivrance du brevet: **13.06.2012**
(21) Numéro de dépôt: 07870392.3
(22) Date de dépôt: 13.12.2007
(51) Int. Cl.: A61B 18/20, A61B 18/18, A61F 9/02

(54) **APPAREIL DE TRAITEMENT PAR EMISSION DE FLASHS LUMINEUX AVEC DISPOSITIF D'ANTI-EBLOUISSEMENT**
LICHTBLITZE AUSSENDENDES BEHANDLUNGSGERÄT MIT BLENDSCHUTZVORRICHTUNG
TREATMENT APPARATUS THAT EMITS LIGHT FLASHES AND INCLUDES AN ANTI-GLARE DEVICE

(30) Priorité: 14.12.2006 FR 0655508
(43) Date de publication de la demande: 23.09.2009
(73) Titulaire: Eurofeedback, 91017 Evry Cedex (FR)
(72) Inventeur: SAFRAOUI, Georges, 91140 Villejust (FR)
(74) Mandataire: Tanty, François
(86) Numéro de dépôt international: PCT/FR2007/052503
(87) Numéro de publication internationale: WO 2008/071898

(56) Documents cités:
- WO-A-2006/089227
- FR-A1- 2 681 443
- US-A- 5 877 825
- US-A1- 2005 177 140

## Description

La présente invention concerne les appareils de traitement par émission de flashs lumineux.

De tels appareils sont utilisés notamment pour des traitements thérapeutiques ou non thérapeutiques, par exemple l'épilation ou les traitements vasculaires ou de rajeunissement.

Ces appareils comportent un poste de base et une pièce à main reliée au poste de base, comportant une lampe flash.

Un organe de déclenchement de l'émission des flashs est prévu sur la pièce à main.

Dans la pratique, le sujet traité porte en général des lunettes partiellement ou complètement opaques, comme par exemple des coquilles métalliques. L'opérateur a besoin de conserver une bonne vision afin de pouvoir effectuer un traitement de qualité. Ce travail peut durer des heures et se répéter tous les jours. Les flashs de lumière incohérente émis par les pièces à main étant réfléchis, ils peuvent à la longue fatiguer l'opérateur.

Une solution peut consister à utiliser des lunettes qui atténuent la lumière sur tout le spectre d'émission. Les pupilles se dilatant pour mieux voir, une telle protection peut toutefois ne pas être entièrement satisfaisante.

En outre, il existe le risque qu'un flash soit émis alors que la pièce à main n'est pas appliquée contre la région à traiter, par exemple en direction du sujet traité.

Pour remédier à ce problème, il a été proposé de munir la pièce à main d'un capteur sensible au contact de la pièce à main contre la région traitée, afin de n'autoriser l'émission d'un flash qu'après détection de ce contact.

Une telle solution rend plus complexe la réalisation mécanique de la pièce à main et ne supprime pas le risque d'éblouissement de l'opérateur par la lumière qui peut être émise par la pièce à main malgré son application contre la région à traiter.

On connaît par ailleurs des lunettes de protection vis-à-vis de l'éblouissement causé par l'émission d'un flash, qui comportent un phototransistor et un obturateur optique qui peut passer d'un premier état optiquement passant à un deuxième état d'atténuation optique en réponse à la détection d'un front lumineux par le phototransistor.

Ces lunettes présentent un temps de réponse entre le moment où le front lumineux est détecté par le phototransistor et celui où l'obturateur passe dans l'état d'atténuation optique qui peut s'avérer trop élevé et ainsi inadapté dans certaines situations, par exemple Lorsqu'une succession de flashs de courte durée est émise par la pièce à main, ce qui est le cas dans certains traitements.

Il existe par conséquent un besoin pour trouver une solution satisfaisante au problème de l'éblouissement causé par l'utilisation des appareils de traitement par émission de flashs lumineux.

L'invention vise à répondre à ce besoin et y parvint grâce à un appareil selon la revendication 1.

Par «système individuel portatif de protection vis-à-vis de l'éblouissement causé par l'émission d'un flash», on désigne tout appareil destiné à protéger les yeux, à savoir des lunettes, une visière, un casque ou un masque.

Par « état optiquement passant », il faut comprendre un état dans lequel l'obturateur permet à l'utilisateur de voir à travers le système de protection. Par « état d'atténuation optique », il faut comprendre un état dans lequel l'atténuation causée par le système de protection permet de réduire l'éblouissement.

La durée d'émission d'un flash suite à la détection de l'action de l'utilisateur sur l'organe de déclenchement peut être inférieure ou égale à 500 ms.

Le poste de base peut être agencé de manière à ce que l'émission d'un flash n'ait lieu qu'après un retard prédéfini, compris par exemple entre 0 et 50 ms, pour permettre le passage du premier état au deuxième état.

En variante, le poste de base peut être agence de manière à ce que l'émission d'un flash ait lieu quelques ms avant le passage du premier état au deuxième état.

Le poste de base peut encore être agencé pour permettre l'émission d'une rafale de flashs à partir de la détection de l'action de l'utilisateur sur l'organe de déclenchement et pour maintenir le ou les obturateur dans le deuxième état pendant au moins la durée de la rafale de flash.

Cette rafale de flashs peut être émise à une fréquence comprise entre 5 et 500 Hz, par exemple.

Dans un exemple de mise en oeuvre de l'invention, le poste de base peut être agencé pour permettre le retour au premier état optiquement passant du ou des obturateurs un court instant avant la fin du flash, ce qui peut permettre de donner à l'opérateur une indication sur le flash émis en toute sécurité.

Selon un mode de réalisation, le système de protection peut comporter au moins deux parties, ce qui peut permettre de l'alléger.

L'appareil peut être agencé pour que la transmission entre le système de protection et le poste de base d'une information commandant le passage du premier étai vers le deuxième état s'effectue par une liaison filaire ou non.

Selon un mode de réalisation, le système portatif de protection individuel comporte un accumulateur et le poste de base peut comporter un logement permettant la réception du système portatif en l'absence d'utilisation et un moyen permettant de recharger l'accumulateur.

Le poste de base peut être agencé pour ne permettre l'émission des flashs qu'après détection d'un signal transmis par le système portatif correspondant au port de celui-ci par l'utilisateur.

Le système portatif de protection individuel peut être agencé pour signaler au poste de base que l'accumulateur est insuffisamment chargé.

Le poste de base peut être agencé pour ne permettre l'émission des flashs qu'après détection d'un signal transmis par le système portatif correspondant à un état de charge suffisant de l'accumulateur.

L'appareil peut être agencé pour empêcher l'émission de flashs lumineux tant que le ou les obturateurs ne sont pas dans le deuxième état.

Le poste de base peut être agencé pour ne permettre l'émission des flashs qu'après détection d'un signal transmis par le système portatif, correspondant au passage du ou des obturateurs dans le deuxième état.

Dans un exemple de mise en oeuvre de l'invention, l'état d'atténuation optique est réglable par l'utilisateur ou de manière automatique, en fonction par exemple du traitement sélectionné par l'utilisateur.

Le poste de base peut être agencé pour adresser au système de protection un signal de réglage de l'intensité d'atténuation ou de la quantité de lumière autorisée par l'ouverture de l'obturateur pendant un temps très court du flash, en début ou en fin de flash, de telle sorte que l'opérateur garde une vision sécurisée des évènements.

L'invention a encore pour objet un procédé de sécurisation du fonctionnement d'un appareil de traitement du corps humain ou animal par émission de flashs lumineux, selon la revendication 15.

L'étape d'émission des flashs lumineux peut être conditionnée à la réception d'un signal provenant du système portatif de protection individuel représentatif du passage effectif du ou des obturateurs dans le deuxième état d'atténuation optique.

Une rafale de flashs peut être émise suite à une action sur l'organe de déclenchement de l'émission des flashs, notamment une rafale de flashs émise à une fréquence comprise entre 5 et 500 Hz.

L'invention pourra être mieux comprise à la lecture de la description détaillée qui va suivre, d'exemples de mise en oeuvre non limitatifs de celle-ci, et du dessin annexé, sur lequel :
- la figure 1 est une représentation schématique d'un premier exemple de réalisation conforme à l'invention,
- les figures 2 et 3 représentent d'autres exemples de réalisation conformes à l'invention, comportant différents systèmes portatifs individuels de protection vis-à-vis de l'éblouissement causé par l'émission d'un flash,
- les figures 4 et 5 sont des chronogrammes illustrant des relations possibles entre différents signaux selon deux exemples de mise en oeuvre de l'invention.

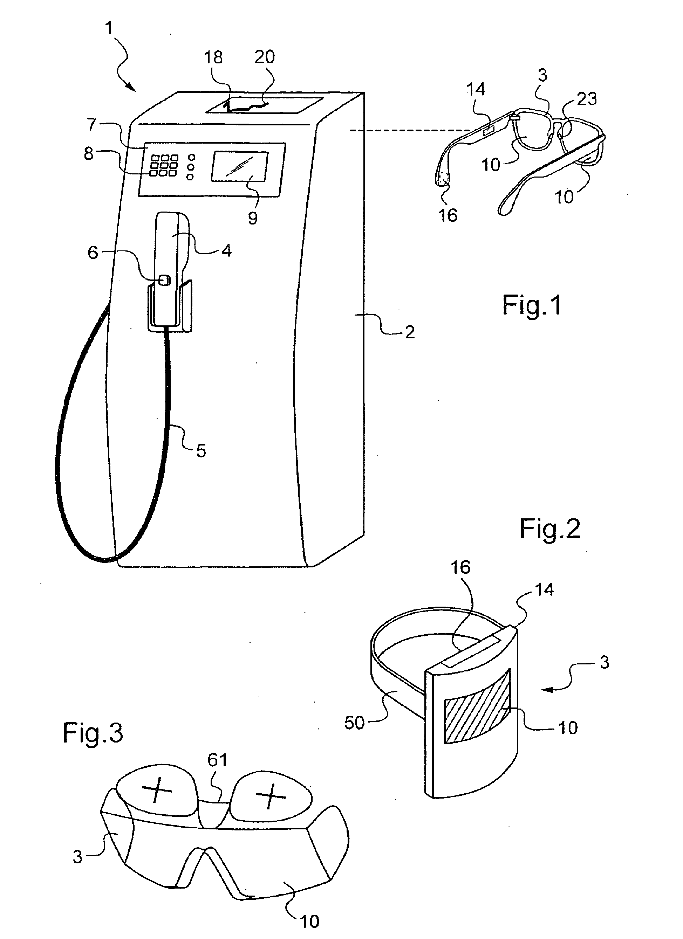

L'appareil 1 représenté à la figure 1 comporte un poste de base 2 et un système 3 portatif individuel de protection vis-à-vis de l'éblouissement causé par l'émission d'un flash.

Ce système 3 se présente, dans l'exemple de la figure 1, sous forme de lunettes 3 destinées à être portées par l'opérateur au cours de l'utilisation de l'appareil.

L'appareil 1 comporte également une pièce à main 4 qui est reliée au poste de base 2 par un câble 5.

La pièce à main 4 comporte un organe 6 de déclenchement de l'émission des flashs lumineux, qui se présente par exemple sous la forme d'un bouton-poussoir sur lequel l'utilisateur appuie pour déclencher l'émission de flashs lumineux.

Ce bouton-poussoir 6 peut être situé en tout emplacement adapté de la pièce à main 4, par exemple sur le dos de celle-ci ou du côté de la face de sortie de la lumière ou ailleurs encore.

L'invention n'est pas limitée à un organe de déclenchement se présentant sous la forme d'un bouton-poussoir et l'organe de déclenchement peut par exemple comporter une touche sensitive ou tout autre interrupteur sensible à une action de l'utilisateur.

La pièce à main 4 intègre au moins un tube flash ainsi qu'un guide optique. Un exemple de pièce à main est décrit dans la demande de brevet FR 2 876 022.

Le poste de base 2 comporte un générateur électrique fournissant l'énergie nécessaire à l'émission des flashs lumineux ainsi qu'un panneau de commande 7 qui comporte par exemple un clavier 8 ou tout autre moyen de sélection permettant à l'utilisateur de sélectionner un type de traitement.

Le panneau de commande 7 peut comporter un écran 9 sur lequel sont affichées des informations permettant de renseigner l'utilisateur sur, par exemple, le traitement sélectionné.

Le poste de base 2 comporte par ailleurs des moyens permettant de transmettre, voire d'échanger, des informations avec le système de protection 3.

Le poste de base 2 peut comporter par exemple des moyens permettant d'établir une liaison sans fil avec le système de protection 3, par exemple une liaison radiofréquence ou infrarouge.

Le poste de base 2 peut ainsi comporter un émetteur radiofréquence permettant de transmettre, de préférence de manière codée, des informations et le système de protection 3 peut comporter un récepteur adapté à recevoir ces informations.

Le système de protection 3 comporte un ou plusieurs obturateurs optiques 10, par exemple deux obturateurs à cristaux liquides placés chacun devant un oeil, qui peuvent passer d'un premier état optiquement passant à un deuxième état d'atténuation optique.

Le système de protection 3 comporte également un circuit électronique 14 qui permet d'une part de recevoir des informations du poste de base 2 et d'autre part de commander le passage du ou des obturateurs 10 du premier état au deuxième état et inversement.

Le circuit électronique 14 peut également être agencé pour transmettre des informations au poste de base 2, comme cela sera précisé plus loin.

Le système de protection 3 peut comporter toute source d'énergie électrique permettant de faire fonctionner le circuit électronique 14, par exemple un accumulateur 16 qui peut être intégré par exemple à l'une des branches dans l'exemple illustré à la figure 1.

Le poste de base 2 peut comporter un logement 18 pour recevoir le système de protection 3 en l'absence d'utilisation.

Le poste de base 2 peut comporter un connecteur 20 qui peut être raccordé au système de protection 3 lorsque celui-ci est en place dans le logement 18, afin de recharger l'accumulateur 16.

Le connecteur 20 peut se présenter sous diverses formes et le système de protection 3 peut présenter tout moyen de raccordement adapté au connecteur 20.

Dans une variante, le rechargement de l'accumulateur 16 s'effectue sans connexion physique, par exemple au moyen d'un couplage inductif, le système de protection 3 comportant par exemple un bobinage permettant de générer une tension électrique lorsqu'exposé à un courant électrique et à un champ magnétique variable généré par un bobinage du poste de base 2.

Dans une variante, le système de protection 3 comporte un logement pour recevoir des piles.

Dans une variante encore, le système de protection 3 est relié par un câble au poste de base 2, ce câble assurant la circulation du courant nécessaire au fonctionnement du ou des obturateurs optiques 10 et permettant éventuellement la transmission d'informations provenant d'un ou plusieurs capteurs présents sur le système de protection 3 vers le poste de base 2.

Dans une variante encore, le système de protection 3 est relié par des fils à un boîtier portatif, par exemple à agrafer à la ceinture ou à mettre dans une poche de vêtement.

Ce boîtier portatif peut comporter le circuit électronique 14 ainsi que l'accumulateur ou toute autre source d'énergie permettant de faire fonctionner le circuit électronique 14. Un tel dispositif peut permettre d'obtenir un système de protection 3 ayant une partie, portée par la tête, plus légère.

Le cas échéant, le système de protection 3 comporte un capteur 23 qui permet de détecter son port par l'utilisateur.

Ce capteur 23 se présente par exemple sous la forme d'un capteur résistif disposé de manière à détecter une variation d'impédance entre deux électrodes lorsque le système de protection 3 est en place sur l'utilisateur.

Le capteur 23 peut encore être un capteur thermique qui permet de détecter une élévation de température locale lorsque le système de protection 3 est porté par l'utilisateur.

D'autres capteurs 23 sont envisageables, par exemple optiques, capacitifs ou mécaniques.

En présence du capteur 23, le système de protection 3 est agencé pour non seulement recevoir des informations du poste de base 2 mais également lui transmettre une information provenant du capteur 23, représentative du port du système de protection 3 par l'utilisateur.

D'autres informations peuvent encore être transmises au poste de base par le système de protection 3, par exemple une information représentative de l'état de charge de l'accumulateur ou de toute autre source d'énergie, ou encore une information représentative du passage effectif du ou des obturateurs optiques 10 dans l'état d'atténuation optique.

Ce passage effectif est par exemple détecté par une cellule photoélectrique.

L'invention n'est pas limitée à une forme particulière de système de protection et dans un exemple de mise en oeuvre, celui-ci se présente sous la forme d'une visière, comme illustré à la figure 2, laquelle peut par exemple ne comporter qu'un seul obturateur 10. Le maintien de cette visière sur la tête de l'utilisateur peut par exemple s'effectuer au moyen d'un serre-tête 50 ou similaire.

La figure 3 représente partiellement une autre variante dans laquelle le système de protection 3 est agencé pour se fixer sur des lunettes 61 existantes, par exemple des lunettes correctrices de vue.

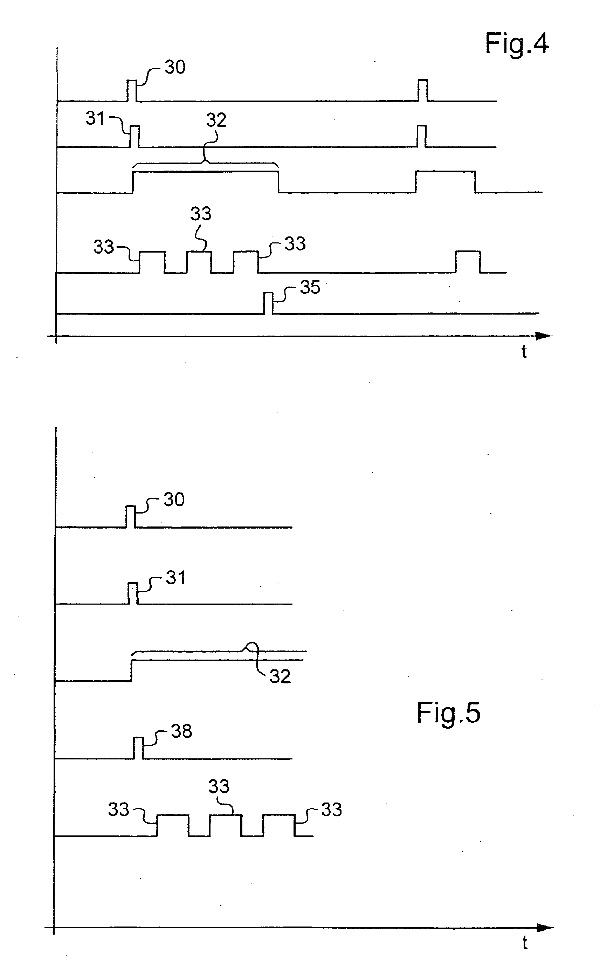

Dans un exemple de mise en oeuvre de l'invention, lorsque l'utilisateur appuie sur l'organe de déclenchement 6, un signal 30 correspondant est généré, comme illustré à la figure 4.

Le poste de base 2 peut être agencé de telle sorte que seul un nombre prédéterminé de flashs soit émis lorsqu'une pression est exercée sur l'organe de déclenchement 6, indépendamment de la durée pendant laquelle cette pression est exercée.

Le signal 30 est détecté par le poste de base 2 qui transmet au système de protection 3 une information de commande 31 de changement d'état du ou des obturateurs 10, de l'état optiquement passant à l'état d'atténuation optique. Ce dernier est assuré par un signal 32 envoyé à ou aux obturateurs 10.

Le circuit électronique 14, à la réception de ce signal de commande 31, provoque le changement de l'état du ou des obturateurs 10 en appliquant la tension correspondante 32 à celui-ci.

Le poste de base 2, après envoi du signal 31 de commande de l'obturation, autorise l'émission des flashs lumineux selon le programme de traitement sélectionné par l'utilisateur. Un signal 33 de commande de l'émission des flashs est ainsi généré, ce signal pouvant comporter plusieurs impulsions correspondant à l'émission d'une rafale de flashs.

Une fois le ou les flashs émis, le poste de base peut envoyer au système de protection 3 un signal 35 provoquant le changement d'état du ou des obturateurs 10 qui retournent à l'état optiquement passant.

Dans la variante de la figure 5, le système de protection 3 est agencé pour échanger des informations avec le poste de base 2 et notamment transmettre à celui-ci un signal 38 représentatif du passage effectif du ou des obturateurs 10 à l'état d'atténuation optique.

Cela peut constituer une sécurité supplémentaire. La poste de base 2 vérifie la réception de cette information 38 avant de générer le signal 33 de commande de l'émission des flashs.

Bien entendu, l'invention n'est pas limitée aux exemples qui viennent d'être décrits.

En particulier, le ou les obturateurs 10 peuvent être de tout type, étant par exemple autres qu'à cristaux liquides, par exemple à diaphragme électromécanique.

Dans une variante de réalisation, l'état d'atténuation optique peut être réglable, par exemple par l'utilisateur, lequel peut par exemple entrer sur le panneau de commande 7 une information permettant de régler le facteur d'atténuation ou le retard désiré avec lequel un flash est émis après le passage du premier au deuxième état de l'obturateur.

Ce réglage peut également se faire de manière automatique en fonction par exemple du traitement sélectionné par l'utilisateur.

L'intensité d'atténuation peut éventuellement encore être réglée sur le système portatif lui-même.

Le cas échéant, un deuxième système de protection peut être utilisé avec le même poste de base, ce système étant porté par le sujet traité.

L'expression « comportant un » doit être comprise comme étant synonyme de « comportant au moins un », sauf si le contraire est spécifié. L'expression « compris entre » s'entend bornes incluses.

## Revendications

1. Appareil (1) de traitement du corps humain ou animal par émission de flashs lumineux, comportant :
- un poste de base (2),
- une pièce à main (4) reliée au poste de base (2), comportant une lampe flash,
- un organe de déclenchement de l'émission des flashs (6),
- un système (3) portatif individuel de protection vis-à-vis de l'éblouissement causé par l'émission d'un flash, comportant un ou plusieurs obturateurs optiques (10) à positionner devant les yeux et pouvant passer d'un premier état optiquement passant à un deuxième état d'atténuation optique,
appareil **caractérisé par le fait qu'**une action de l'utilisateur sur l'organe de déclenchement (6) est détectée par le poste de base (2) et dans lequel le passage du premier état au deuxième état s'effectue en réponse à la détection de cette action, et
**par le fait que** le système de protection (3) comporte un capteur (23) agencé pour détecter le port du système par l'utilisateur, le système (3) étant agencé pour informer le poste de base lorsque le capteur (23) détecte que le système portatif (3) est porté par l'utilisateur.

2. Appareil selon la revendication 1, le poste de base (2) étant agencé de manière à ce que l'émission d'un flash n'ait lieu qu'après un retard compris notamment entre 0 et 50 ms pour permettre le passage du premier état au deuxième état.

3. Appareil selon la revendication 1, le poste de base (2) étant agencé de manière à ce que l'émission d'un flash ait lieu avant le passage du premier état au deuxième état, notamment quelques ms avant.

4. Appareil selon l'une quelconque des revendications précédentes, le poste de base (2) étant agencé pour permettre le retour au premier état optiquement passant du ou des obturateurs (10) avant la fin du flash.

5. Appareil selon l'une quelconque des revendications 1 à 3, le poste de base (2) étant agencé pour permettre l'émission d'une rafale de flashs à partir de la détection de l'action de l'utilisateur sur l'organe de déclenchement (6) et pour maintenir le ou les obturateurs dans le deuxième état pendant au moins la durée de la rafale de flashs.

6. Appareil selon l'une quelconque des revendications 1 à 3, le poste de base (2) étant agencé de telle sorte que seul un nombre prédéterminé de flashs soit émis lorsque l'utilisateur agit sur l'organe de déclenchement (6), indépendamment de la durée de cette action.

7. Appareil selon la revendication précédente, la rafale de flashs étant émise à une fréquence comprise entre 5 et 500 Hz.

8. Appareil selon l'une quelconque des revendications précédentes, la durée d'émission d'un flash suite à la détection de l'action de l'utilisateur sur l'organe de déclenchement (6) étant inférieure ou égale à 500 ms.

9. Appareil selon l'une quelconque des revendications précédentes, étant agencé pour transmettre entre le système de protection et le poste de base une information commandant le passage du premier état vers le deuxième état par une liaison sans fil.

10. Appareil selon l'une quelconque des revendications précédentes, l'état d'atténuation optique étant réglable par l'utilisateur sur le poste de base (2) ou sur le système (3) portatif, ou de manière automatique.

11. Appareil selon l'une quelconque des revendications précédentes, un deuxième système de protection étant utilisé avec le même poste de base (2), ce système étant porté par le sujet traité.

12. Appareil selon l'une quelconque des revendications précédentes, le poste de base (2) étant agencé pour adresser au système (3) de protection un signal de réglage de l'intensité d'atténuation ou de la quantité de lumière autorisée par l'ouverture de l'obturateur (10) pendant un temps très court du flash, en début ou en fin de flash.

13. Appareil selon l'une quelconque des revendications précédentes, l'appareil étant agencé pour empêcher l'émission de flashs lumineux tant que le ou les obturateurs (10) ne sont pas dans le deuxième état.

14. Appareil selon l'une quelconque des revendications précédentes, le capteur (23) se présentant sous la forme d'un capteur résistif disposé de manière à détecter une variation d'impédance entre deux électrodes lorsque le système de protection (3) est en place sur l'utilisateur, ou étant un capteur thermique permettant de détecter une élévation de température locale, ou étant un capteur optique, capacitif ou mécanique.

15. Procédé de sécurisation du fonctionnement d'un appareil (1) de traitement du corps humain ou animal par émission de flashs lumineux, comportant les étapes successives consistant à:
- détecter une action exercée par un opérateur sur un organe de déclenchement de l'émission des flashs (6), et
- amener un ou plusieurs obturateurs optiques (10) d'un système portatif individuel de protection (3), disposé devant les yeux de l'opérateur, à passer d'un premier état optiquement passant à un deuxième état d'atténuation optique, procédé **caractérisé par le fait qu'**il comporte en outre les étapes suivantes :
- détecter le port effectif du système portatif de protection individuel (3), et émettre un message en cas de non détection du port dudit système et/ou empêcher l'émission des flashs lumineux, et
- autoriser l'émission d'un ou plusieurs flashs lumineux.

## Claims

1. apparatus (1) for treating the human or animal body by emission of light flashes, comprising:
- a base station (2),
- a handpiece (4) connected to the base station (2), comprising a flash lamp,
- a member for triggering the emission of the flashes (6),
- a personal portable System (3) for protection against glare caused by the emission of a flash, comprising one or more optical occluders (10) to be positioned in front of the eyes and being le to switch from a first optically transparent state to a second optical attenuation state,
apparatus **characterized by** the fact that an action of the user on the triggering member (6) is detected by the base station (2) and in which the transition from the first state to the second state takes place in response to the detection of this action, and
by the fact that the protection system (3) comprises a sensor (23) arranged to detect the wearing of the system by the user, the system (3) being arranged to inform the base station when the sensor (23) detects that the portable system (3) is being worn by the user.

2. The apparatus as claimed in claim 1, the base station (2) being arranged so that the emission of a flash takes lace only after a delay ranging notably between 0 and 50 ms in order to allow the transition from the first state to the second state.

3. The apparatus as claimed in claim 1, the base station (2) being arranged so that the emission of a flash takes lace before the transition from the first state to the second state, notably a few ms beforehand.

4. The apparatus as claimed in any one of the preceding claims, the base station (2) being arranged to allow the return to the first optically transparent state of the occluder(s) (10) before the end of the flash.

5. The apparatus as claimed in any one of claims 1 to 3, the base station (2) being arranged to allow the emission of a burst of flashes starting from the detection of the action of the user on the triggering member (6) and in order to keep the occluder(s) in the second state for at least the duration of the burst of flashes.

6. The apparatus claimed in any one of claims 1 to 3, the base station (2) being arranged so that only a predetermined number of flashes is emitted when the user makes an action on the triggering member (6), irrespective of the duration of this action.

7. The apparatus as claimed in the preceding claim, the burst of flashes being emitted at a frequency of between 5 and 500 Hz.

8. The apparatus as claimed in any one of the preceding claims, the duration of emission of a flash following the detection of the action of the user on the triggering member (6) being less than or equal to 500 ms.

9. The apparatus as claimed in any one of the preceding claims, being arranged in order to transmit between the protection system and the base station an information commanding the transition from the first state to the second state by a wireless link.

10. The apparatus as claimed in any one of the preceding claims, the optical attenuation state being adjusted by the user on the base station (2) or on the ortable system (3), or automats

11. The apparatus as claimed in any one of the preceding claims, a second protection system being used with the same base station (2), this system being worn by the treated subject.

12. The apparatus as claimed in any one of the preceding claims, the base station (2) being arranged to send to the protection system (3) a signal for adjusting the intensity of attenuation or the quantity of light authorized by the opening of the occluder (10) for a very short time of the flash, at the beginning or at the end of the flash.

13. The apparatus as claimed in any one of the preceding claims, the apparatus being arranged to prevent the emission of light flashes while the occluder(s) (10) is/are not in the second state.

14. The apparatus as claimed in any one of the preceding claims, the sensor (23) taking the form of a resistive sensor placed so as to detect a variation of impedance between two electrodes when the protection system (3) is in lace on the user, or being a heat sensor which makes it possible to detect a local rise in temperature, or being an optical, capacitive or mechanical sensor.

15. A protection method for the operation of an apparatus (1) for treating the human or animal body by emission of light flashes, comprising the successive steps consisting in:
- detecting an action taken by an operator on a member for triggering the emission of the flashes (6), and
- causing one or more optical occluders (10) of a personal portable protection system (3), lace in front of the eyes of the operator, to switch from a first optically transparent state to a second optical attenuation state,
method **characterized by** the fact that it furthermore comprises the following steps:
- detect the actual wearing of the personal portable protection system (3), and emit a message if the wearing of said system is not detected and/or prevent the emission of the light flashes, and
- authorizing the emission of one or more light flashes.

## Patentansprüche

1. Vorrichtung (1) zur des menschlichen oder tierischen Körpers durch Emission von Lichtblitzen, mit:
- einer Basisstation (2),
- einem mit der Basisstation (2) verbundenen (4), das eine Blitzlampe ausweist,
- einem Organ (6) zum der Lichtblitze,
- einem persönlichen tragbaren System (3) zum schutz gegen Blendung, die durch die eines Blitzes verursacht wird, not einer er mehreren optischen Blenden (10), die vor den Augen zu positionieren sind und von einem ersten, optisch durchlässigen Zustand in einen zweiten, optisch dämpfenden Zustand übergehen können,
welche Vorrichtung **dadurch gekennzeichnet ist, dass** eine Betätigung des Auslöseorgans (6) durch den Benutzer von der Basisstation (2) detektiert und in welcher der Übergang von dem ersten Zustand in den zweiten Zustand als Reaktion auf die die on dieser Betätigung stattfindet,
sowie dadurch, dass das Schutzsystem (3) einen Sensor (23) der dazu ausgebildet ist das Tragen des Systems durch den Benutzer zu detektieren, wobei das System (3) dazu ausgebildet ist, die Basisstation zu informiereren wenn der Sensor (23) detektiert, dass das tragbare System (3) von dem Benutzer getragen wird.

2. Vorrichtung nach Anspruch 1, bei der die Basisstation (2) so ist, dass die Emission eines Blitzes erst nach einer Verzögerungszeit stattfindet, insbesondere zwischen 0 und 50 ms liegt, um den Übergang aus dem ersten Zustand den zweiten Zustand zu ermöglichen.

3. Vorrichtung nach anspruch 1, bei der die Basisstation so ausgebildet ist, dass die Emission eines Blitzes vor dem Übergang aus dem ersten Zustand in den zweiten Zustand stattfindet, insbesondere einige ms vorher.

4. Vorrichtung nach einem der vorstehenden Ansprüche, bei der die Basisstation dazu ausgebildet ist, vor dem Ende des Blitzes die Rückkehr der Blende oder Blenden (10) in den ersten, optisch durchlässigen Zustand zu erlauben.

5. Vorrichtung nach der Ansprüche 1 bis 3, bei der die Basisstation (2) dazu ausgebildet ist, nach der Detektion der Betätigung des Auslöseorgans (6) durch den Benutzer die Emission einer Serie von Blitzen zu erlauben und die Blende oder Blenden zumindest während der Dauer der Serie der Blitze in dem zweiten Zustand zu halten.

6. Vorrichtung nach einem der Ansprüche 1 bis 3, bei der die Basisstation (2) so ausgebildet ist, dass nur eine vorbestimmte Anzahl von Blitzen emittiert wird, wenn der Benutzer das Auslöseorgan (6) betätigt, unabhängig von der Dauer dieser Betätigung.

7. Vorrichtung nach dem vorstehenden Anspruch, bei der die Serie der emittierten Blitze eine Frequenz zwischen 5 und 500 Hz hat.

8. Vorrichtung nach einem der vorstehenden Ansprüche, bei der die Dauer der Emission eines Blitzes im Anschluss an die Detektion der Betätigung des Auslöseorgans (6) durch den Benutzer kleiner oder gleich 500 ms ist.

9. Vorrichtung nach einem der vorstehenden Ansprüche, die dazu ausgebildet ist, zwischen dem Schutzsystem und der Basisstation über eine drahtlose Verbindung eine Information zu übertragen, die den Übergang aus dem ersten Zustand in den zweiten Zustand befiehlt.

10. Vorrichtung nach einem der vorstehenden Ansprüche, bei der der dämpfende Zustand von dem Benutzer an der Basisstation (2) oder an dem tragbaren System (3) oder automatisch einstellbar ist.

11. Vorrichtung nach einem der vorstehenden Ansprüche, bei der ein zweites Schutzsystem mit derselben Basistation (2) verwendet wird, wobei dieses System von dem behandelten Objekt getragen wird.

12. Vorrichtung nach einem der vorstehenden Ansprüche, bei der die Basisstation (2) dazu ausgebildet ist, an das Schutzsystem (3) ein Signal zur Einstellung der Stärke der Dämpfung zu senden oder zur Einstellung der zugelassenen Lichtmenge durch Öffnen der Blende (10) während einer sehr kurzen des Blitzes am Beginn oder am Ende des Blitzes.

13. Vorrichtung nach einem der vorstehenden Ansprüche, die dazu ausgebildet ist, die Emission von Lichtblitzen zu verhindern, während die Blende oder Blenden (10) sich nicht in dem zweiten Zustand befinden.

14. Vorrichtung nach einem der vorstehenden Ansprüche, bei der der Sensor (23) die Form eines Widerstandssensors hat, der so angeordnet ist, dass er eine Änderung der Impedanz zwischen zwei Elektroden detektiert, wenn das Schutzsystem (3) an dem Benutzer in Position ist, oder die Form eines thermischen Sensors, der es erlaubt, eine lokale Erhöhung der Temperatur zu detektieren, oder die Form eines optischen, kapazitiven oder mechanischen Sensors.

15. Verfahren zur Sicherung der Funktion einer Vorrichtung (1) zur Behandlung des menschlichen oder tierischen Körpers durch Emission von Lichtblitzen, mit den aufeinanderfolgenden Schritten, die bestehen in:
- Detektieren einer Betätigung eines Auslöseorgans (6) für Lichtblitze durch einen Bediener und
- Überführen einer oder mehrerer optischer Blenden (10) eines tragbaren persönlichen Schutzsystems (3), die vor den Augen des Bedieners angeordnet sind, aus einem ersten, optisch durchlässigen Zustand in einen zweiten, optisch dämpfenden Zustand, welches Verfahren **dadurch gekennzeichnet ist, dass** es außerdem die folgenden Schritte aufweist:
- Detektieren, dass das persönliche tragbare Schutzsystem (3) tatsächlich getragen wird, und Emittieren einer Nachricht in dem Fall der Nicht-Detektion des Tragens des genannten Systems und/oder Verhindern der Emission von Lichtblitzen und
- Zulassen der Emission eines oder mehrerer Lichtblitze.
